# EUROPEAN PATENT APPLICATION

(11) **EP 4 760 742 A2**
(43) Date of publication of application: **17.06.2026**
(21) Application number: 26158722.4
(22) Date of filing: 26.05.2022
(51) Int. Cl.: G16H 40/60

(54) **AN INTELLIGENT PUMP-BASED FLUID COLLECTION SYSTEM AND RELATED METHODS**

(30) Priority: 27.05.2021 US 202163193891 P
(62) Divisional of application: 22732368.0
(71) Applicant: Purewick Corporation, El Cajon, California 92020 (US)
(72) Inventor: VAN GORKOM, Aaron, Lynnwood, Washington 98036 (US); GARVEY, Tony, Listowel, V31CK75 (IE); CARRACIOLA, Meghan, Washington, District of Columbia 20003 (US); FOGARTY, Padraig, Tipperary, V94K5P5 (IE); RICHARDSON, Tamika, Schenectady, New York 12306 (US)
(74) Representative: Hoffmann Eitle

(57) **Abstract**

Examples relate to devices, systems, and methods for collecting discharged fluids. A fluid collection system includes a fluid collection device configured to collect fluid discharged from a user and a fluid collection container configured to receive the fluid from the fluid collection device. The fluid collection system also includes a pump configured to pull an at least partial vacuum to draw the fluid from the fluid collection device and into the fluid collection container. The fluid collection system also includes a control system including at least one sensor configured to monitor at least one of a property or status of the fluid collection system and a controller operatively coupled to the at least one sensor and configured to communicate at least one of the property or the status of the fluid collection system to a control panel.

## Description

### CROSS-REFERENCE TO RELATED APPLICATIONS

This application claims priority to U.S. Provisional Patent Application No. 63/193,891 filed on May 27, 2021, the disclosure of which is incorporated herein, in its entirety, by this reference.

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated with their use. For example, bed pans may be prone to discomfort, spills, and other hygiene issues. Urinary catheters be may be uncomfortable, painful, and may cause urinary tract infections.

Thus, users and manufacturers of fluid collection devices continue to seek new and improved devices, systems, and methods to collect bodily fluids.

### SUMMARY

Embodiments disclosed herein are related to fluid collection devices and methods of using fluid collection devices. In an embodiment, a fluid collection system may include a fluid collection device configured to collect fluid discharged from a user and a fluid collection container configured to receive the fluid from the fluid collection device. The fluid collection system may also include a pump in fluid communication with the fluid collection device and the fluid collection container. The pump may be configured to pull an at least partial vacuum to draw the fluid from the fluid collection device and into the fluid collection container. A control system including at least one sensor configured to monitor at least one of a property or status of the fluid collection system and a controller operatively coupled to the at least one sensor configured to communicate at least one of the property or the status of the fluid collection system to a control panel.

In an embodiment, a method of using a fluid collection system may include placing a fluid collection device at least proximate to a urethra of a user and receiving fluid discharged from the user in a fluid collection device. The method further includes receiving fluid discharged from the fluid collection device in a fluid collection container. The method also includes determining, with at least one sensor, at least at least one of a property or status of the fluid collection system. The method also includes transmitting, from a control system, to a control panel the at least one of the property or the status of the fluid collection system.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** is a block diagram of a fluid collection system, according to an embodiment.
**FIG. 1B** is an isometric view of a fluid collection system, according to an embodiment.
**FIG. 2** is a block diagram of a fluid collection system, according to an embodiment.
**FIG. 3** **is** a schematic for a controller of a fluid collection system, according to an embodiment.
**FIG. 4A** is a schematic view of a control panel with a display indicating a property or status of the fluid collection system, according to an embodiment.
**FIG. 4B** is a schematic view of a control panel with a display indicating a property or status of the fluid collection system, according to an embodiment.
**FIG. 5** is a flow diagram of a method of using a fluid collection system, according to an embodiment.

### DETAILED DESCRIPTION

Embodiments disclosed herein are related to fluid collection systems including at least one sensor at least one of a property or status of the fluid collection system, and methods of using the same. The devices and systems disclosed herein are configured to collect fluids from an individual and include a fluid collection device, a fluid collection container configured to receive the fluid from the fluid collection device, and pump in fluid communication with the fluid collection device and the fluid collection container. The fluids collected by the fluid collection devices may include at least urine or other bodily fluids. Embodiments of fluid collection systems disclosed herein may include a controller operatively coupled to the at least one sensor that communicates at least one of the property or the status of the fluid collection system to a control panel. The at least one property of the fluid collection system may be related at least to a volume of the fluid within the fluid collection container.

In some embodiments, the property may include at least one of a volume or a mass of the fluid in the fluid collection container. At least one of the means for sensing may include a sensor disposed adjacent to an interior portion of the fluid collection container, wherein the sensor is a capacitive sensor configured to detect a property related to the volume or mass of the fluid in the fluid collection container.

The fluid collection system also may include a controller operatively coupled to the at least one sensor and configured to communicate at least one of the property or the status of the fluid collection system to a control panel. By sensing at least one of a property or status of the fluid collection system and having a controller that communicates at least one of the property or status of the fluid collection system to the control panel, more accurate information and/or status of the user and/or the fluid collection system may be determined, collected, and/or utilized in real-time.

In some examples, the computing device or system receiving at least one of the detected flow property or the volume of the fluid may include a module attached to an arm of a wheelchair or a smartphone. As shall be described in greater herein, the computing device may include a programmable timer, an alert or alarm, or a display configured to display the at least one property of the fluid or the status of the fluid collection system. In some examples, the computing device may be wirelessly coupled to the controller.

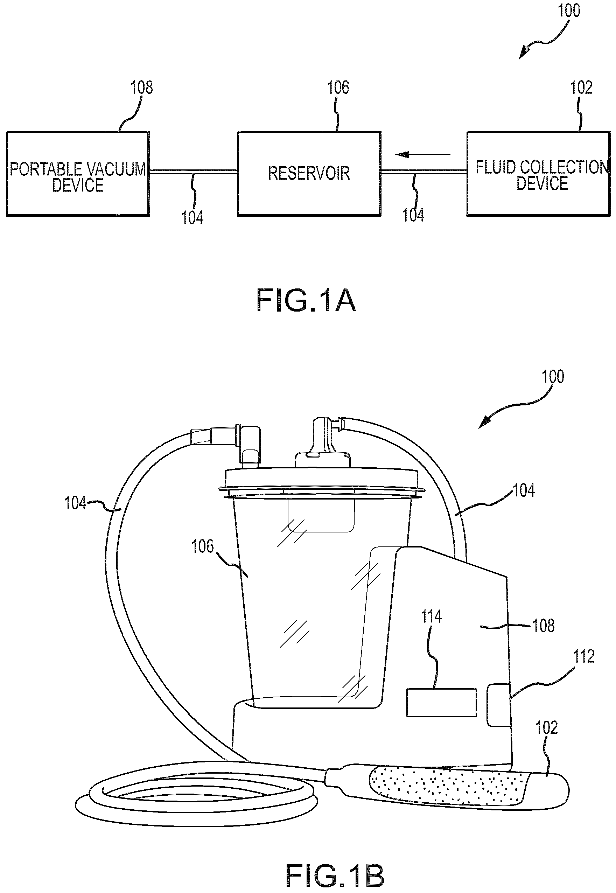

**FIG. 1A** is a block diagram of a fluid collection system 100, according to an embodiment. The fluid collection system 100 includes a fluid (e.g., urine) collection device 102 configured to collect fluid discharged from a user. The fluid collection device 102 may include a male or female collection device. PCT International Application No. PCT/US2019/029616, for example, describes various embodiments of both male and female fluid collection devices, the disclosure of which is incorporated, in its entirety, by this reference. Moreover, the fluid collection device 102 may be interchangeable in the fluid collection system 100 between different types, varieties, and sizes of male or female fluid collection devices. The fluid collection device 102 may be configured to be positioned proximate or adjacent to a urethra of a user. Generally, the fluid collection device 102 may include a surface sized to be positioned proximate or adjacent to the urethra and configured to wick fluid or other fluids away from the user. Urine or other fluids may be wicked from the surface to a reservoir in the fluid collection device 102.

In some embodiments, the fluid collection system 100 may include a tube 104 that couples the fluid collection device 102 to a fluid collection container 106 and a vacuum device or pump 108. The tube 104 may include a flexible material such as plastic tubing (e.g., medical tubing). Such plastic tubing may include a thermoplastic elastomer, polyvinyl chloride, ethylene vinyl acetate, polytetrafluoroethylene, etc., tubing. In some embodiments, the tube 104 may include silicone or latex. In other embodiments, the tube 104 may include a reinforced tube having metal or other electrically conductive components therein. The fluid collection device 102, the container 106, and the pump 108 may be fluidly coupled to each other via one or more tubes 104. For example, fluid collection device 102 may be operably coupled to one or more of the fluid collection container 106 or the pump 108 via the tube 104. Fluid (e.g., urine or other bodily fluids) collected in the fluid collection device 102 may be removed from the fluid collection device 102 via the tube 104 coupled to the fluid collection device 102. A vacuum may be introduced into an interior chamber of the fluid collection device 102 via an inlet of the tube 104 responsive to suction (e.g., vacuum) force applied at an outlet of the tube 104.

The vacuum or suction force may be applied to the tube 104 by the pump 108 either directly or indirectly. The vacuum may be applied indirectly via the fluid collection container 106. For example, the outlet of the tube 104 may be connected to or disposed within the fluid collection container 106 and an additional tube 104 may extend from the fluid collection container 106 to the pump 108 or other suitable vacuum device. Accordingly, the pump 108 may apply a suction to the fluid collection device 102 via the fluid collection container 106. The vacuum may be applied directly via the pump 108. For example, the outlet of the tube 104 may be disposed within the pump 108. An additional tube 104 may extend from the pump 108 to a point outside of the fluid collection device 102, such as to the fluid collection container 106. In other embodiments, the pump 108 may be coupled to an exterior of the tube 104 to draw vacuum. In such examples, the pump 108 or other vacuum device may be disposed between the fluid collection device 102 and the fluid collection container 106.

The pump 108 may include one or more of a manual vacuum pump, and electric vacuum pump, a diaphragm pump, a centrifugal pump, a displacement pump, a magnetically driven pump, a peristaltic pump, or any pump configured to produce a vacuum. The pump 108 may provide a vacuum or suction to remove fluid from the fluid collection device 102 that may be discharged from the user. In some examples, the pump 108 may be powered by one or more of a power cord (e.g., connected to a power socket), one or more batteries, or even manual power *(e.g.,* a hand operated vacuum pump). In some examples, the pump 108 may be sized and shaped to fit outside of, on, or within the fluid collection device 102. For example, the pump 108 may include one or more miniaturized pumps or one or more micro pumps.

Referring now to **FIG. 1B****,** in some embodiments, the fluid collection container 106 may be sized and shaped to retain a fluid therein. The fluid collection container 106 may include a bag (*e.g.,* drainage bag), a bottle or cup (*e.g.,* collection jar), or any other enclosed container for storing bodily fluid(s) such as urine. In some embodiments, the fluid collection container 106 may include a generally rigid exterior housing an interior portion configured to contain the fluid. In some examples, the tube 104 may extend from the fluid collection device 102 and attach to the fluid collection container 106 at a first point therein. An additional tube 104 may couple to the fluid collection container 106 at a second point therein and may extend and attach to the pump 108. Accordingly, the pump 108 may be configured to pull an at least partial vacuum to draw the fluid from the fluid collection device 102 through the tube 104 into the fluid collection container 106. Fluid, such as urine, may be drained from the fluid collection device 102 and into the fluid collection container 106 via the pump 108.

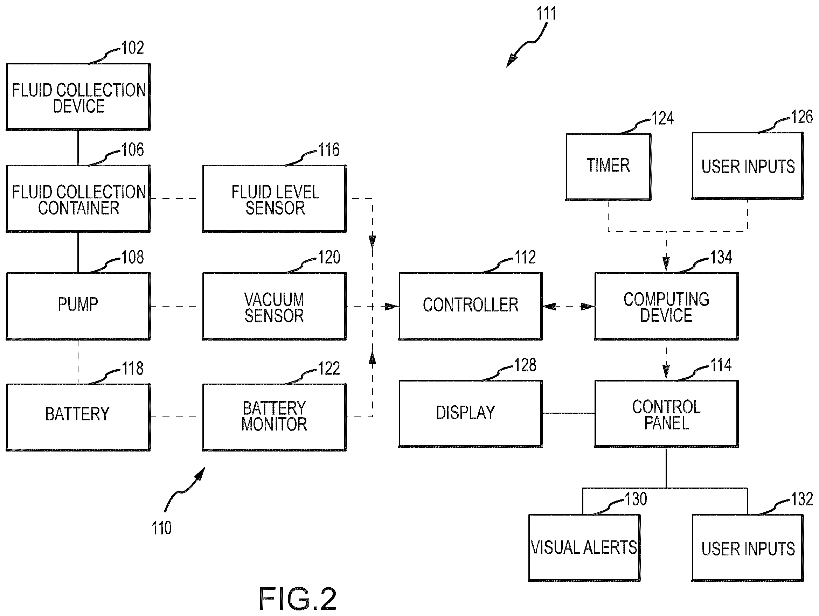

The fluid collection system 100 may include at least one sensor 110 (not shown in FIG. 1B, see FIG. 2) configured to monitor at least one of a property or status of the fluid collection system 100. The at least one sensor may include a fluid level sensor disposed adjacent to the fluid collection container 106, a battery sensor, and/or a power consumption sensor operatively coupled to the pump. The fluid collection system 100 may also include a control system 111 that includes a controller 112 operatively coupled to the at least one sensor that communicates at least one of the property or status of the fluid collection system 100 to a control panel 114. Additional details regarding the schematics, power supply, and communications of the at least one sensor 110, controller 112 and the control panel 114 are provided below in reference to **FIG. 2****.**

In some embodiments, the controller 112 may be integrated with the control panel 114. In some embodiments, the sensor 110 may be configured to communicate the property of the fluid to the controller 112 which may then communicate the property of the fluid to the control panel 114 which may include a software application configured to record data including an amount of fluid received or drank by the user, display the volume of the fluid discharged by the user, and/or determine and display trends and totals related to the volume of the fluid discharged by the user. The control panel 114 may be located on a housing for the pump 108 or on a remote controller (not shown). In some examples, the pump 108, the at least one sensor 110, the controller 112, and the control panel 114 may be powered by one or more batteries 118.

In some embodiments, the control panel 114 can be remotely operated. The control panel 114 can be at least a portion of the remote controller. In some embodiments, one or more touch sensitive displays and/or buttons may be provided on the device. For example, the remote controller may include a touch screen that occupies a portion the front face of the device. The remote controller can also include also include indicator lights and/or display to show a property or status of the fluid collection system 100. In some embodiments, the remote controller can include a physical connection to the fluid collection container 106, such as an electrical wire, or can be a wireless connection, such as Bluetooth, Wi-Fi, proximity sensors, etc. For example, the remote controller can include a fob that allows a user in a wheelchair to interact and/or control the fluid collection system 100 when the fluid collection container 106 is disposed in a bag and/or hanging on the wheelchair or otherwise inaccessible. The seated user could view system status via visual LEDs and/or other indicators and effect control of the fluid collection system from the remote control and/or fob. In some examples, the remote controller can be a personal computing device such as a smart phone, a smart watch, a tablet, or any other form of electronic device.

Referring now to **FIG. 2****,** in some embodiments, the control system 111 may include at least one sensor 110. In some embodiments, the control system 111 may include one sensor 110 or more than one sensor 110. The sensors shown in FIG. 2 may be configured to be the only sensor or may be used in combination with other sensors. Each of the sensors shown in FIG. 2 may be optional to include in the fluid collection system 100.

The at least one sensor 110 may include a fluid level sensor 116 disposed within and/or adjacent to the fluid collection container 106. The fluid level sensor 116 may be configured to detect a property related to the volume or mass of the fluid in the fluid collection container 106. In some embodiments, the fluid level sensor 116 may include a capacitive sensor. The fluid level sensor 116 may include two conducting plates or electrodes. When an electric charge is applied to the conducting plates, the space in between the plates will also charge. The charge amplitude between the two plates depends on the dielectric. When the fluid level is below the sensor, the dielectric is air and when the fluid collection container 106 fills with fluid, because the fluid has a higher capacitance than the air, the capacitance of the fluid level sensor 116 rises approximately linearly with the fluid level in the fluid collection container 106. In some embodiments, the fluid level sensor 116 may be located along a wall of the fluid collection container 106.

In some embodiments, the at least one sensor 110 may include a vacuum sensor 120 operatively coupled to the pump 108. The vacuum sensor 120 may include a sealed piston/Cylinder to measure changes in pressure. Mechanical deflection uses an elastic or flexible element to mechanically deflect with a change in pressure, for example a diaphragm, Bourdon tube, or bellows. In other embodiments, the vacuum sensor 120 may include a piezoelectric pressure sensor that measures dynamic and quasi-static pressures. The bi-directional transducers consist of metalized quartz or ceramic materials that have naturally occurring electrical properties. They are capable of converting stress into an electric potential and vice versa. In other embodiments, the vacuum sensor 120 may include a variable capacitance pressure instrument. The capacitance pressure instrument may use the capacitance change results from the movement of a diaphragm element to measure pressure. The capacitance pressure instrument may use a thin diaphragm as one plate of a capacitor. The applied pressure causes the diaphragm to deflect and the capacitance to change. The deflection of the diaphragm may cause a change in capacitance that is detected by a bridge circuit. Capacitive absolute pressure sensors with a vacuum between the plates may prevent error by keeping the dielectric constant of the material constant. Other suitable vacuum sensors 120 may be included. The vacuum sensor 120 may provide data about the pump speed and lifetime of the pump. In some embodiments, the vacuum sensor 120 may provide information as to when the pump is pumping urine or air. In some embodiments, the vacuum sensor may provide information as to the effectiveness of the pump 108.

In some embodiments, the control system 111 may control the operations of the pump 108. The control system 111 may be configured to start and stop the pump. The control system may start and/or stop the pump according to a predetermined schedule. The control system 111 may start the pump when the fluid level sensor 116 reaches a predetermined limit and stop the pump when fluid collection container 106 is empty or the urine level is below a predetermined threshold. In some embodiments, the control system 111 may increase or decrease power to the pump 108 and/or adjust the speed of the pump 108 when the pump 108 includes a variable speed pump.

In some embodiments, the at least one sensor 110 may include a battery monitor 122. The number of cycles it has to perform and the depth of the discharge determine the life of the battery 118. In general, the optimum life to utility ratio will occur if the battery is not discharged lower than 50%. An amp hour meter counts the rate and time of current flow from a battery being discharged and does the same when the battery is being charged back up. Once the batter monitor 122 determines the full point, the capacity and the efficiency of the battery the amp hour meter is in a position to give an instantaneous readout of the state of charge in amp hours or percent of total at any time in the cycle. In some embodiments, the battery monitor may include an impedance-based meter to measure the state of charge of the battery 118. The impedance-based meter measures battery impedance and by referring to stored data uses the impedance to produce a reading of how full the battery is. The impedance-based meter may provide battery voltage and percentage charged.

In some embodiments, the control system 111 may include a timer 124 and/or various user inputs 126. For example, the timer 124 may include an input from the user or caregiver to replace a component of the fluid collection system 100, empty the fluid collection container 106, or start or stop the pump 108. The timer 124 may be included to determine the useable life of the battery 118, the fluid collection device 102, the pump 108, the tube 104, and/or any other components of the fluid collection system 100. The timer 124 may indicate predictive maintenance procedures. Notifications and/or reminders may indicate replacement intervals of components, including filters and batteries. User inputs 126 may include switches and/or control buttons to control pump status, power to the fluid collection system 100, pump speeds, setting timers and acknowledging alarms, and or other suitable inputs.

The control system 111 may also include indicator lights and/or display 128. In some embodiments, the display may include an alphanumeric display. In other embodiments, the display 128 may include light-emitting diode (LED) lights. The display 128 may include a liquid crystal display (LCD), a thin-film transistor (TFT) display, an OLED display, AMOLED display, and/or a QLED display. In some embodiments, visual alerts 130 and audible alerts 132 may be included. The alerts 130, 132 may indicate a high urine level in the container 106, an electrical fault or other condition with the controller 112, the timer 124 response, a low battery, replacement indication for components of the fluid collection system 100, and/or a failed or failing pump condition.

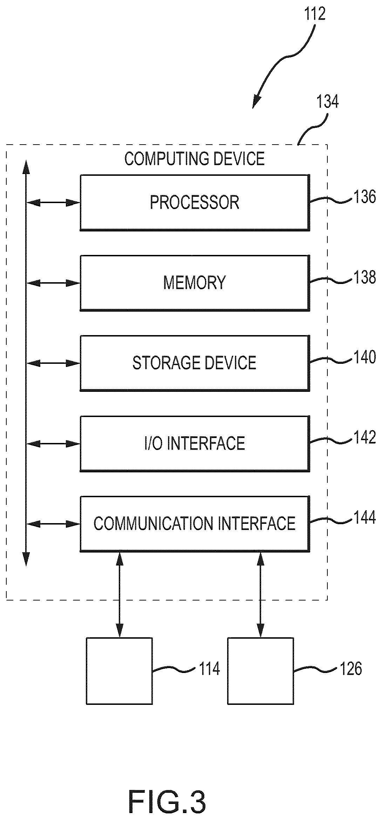

**FIG. 3** is a schematic of controller 112 that may be integrated into the at least one sensor 110 discussed above or the computing device 134 described herein, according to some embodiments. The controller 112 may be configured to communicate with any of the at least one sensor 110, such as with a wired or wireless connection. In some embodiments, the at least one sensor 110 may include the controller 112. In an embodiment, the controller 112 may include a printed circuit board (PCB) equipped with erasable programmable read-only memory (EPROM) for memory of at least data collected by the at least one sensor 110. For example, the controller 112 may be configured to calculate a level or volume of fluid in the fluid collection container 106 or the battery discharge rate. The controller 112 may be configured to send notifications or alerts to other electronic devices. For example, the controller 112 may be configured to send notifications or alerts to the control panel 114. An external or internal battery, such as a rechargeable or a single use battery may power the controller 112.

In some embodiments, the controller 112 may be configured to transmit an alert to the control panel 114 relating at least to the status of the pump 108. In some embodiments, the controller 112 may be configured to operate and/or control the pump 108. In some embodiments, the controller 112 may control the pump 108 based off the status of the volume of fluid detected by the fluid level sensor 116 in the fluid collection container 106. For example, based on data from the at least one sensor 110, the controller 112 may start the pump 108 to cause fluid to flow from the fluid collection device 102 to the fluid collection container 106. The controller 112 may cause the pump 108 to secure when the volume of fluid in the fluid collection container 106 reaches a predetermined threshold. In some embodiments, the controller 112 may wirelessly transmit alerts and selected frequencies, such as selected time and/or volume intervals. The controller 112 may communicate to the control panel 114 when the battery 118 is low. The controller 112 may transmit an alert to the module 140 and/or the smartphone 142 of the user or the caregiver when the pump 108 or fluid collection system 100 has a malfunction. In some embodiments, the controller 112 may be configured to control all operations of the pump 108.

The controller 112 may include at least one computing device 134, according to an embodiment. The at least one computing device 134 may be an exemplary computing device that may be configured to perform one or more of the acts described above. The computing device 134 may include at least one processor 136, memory 138, a storage device 140, an input/output ("I/O") device/interface 142, and a communication interface 144.

In some examples, the processor 136 may include hardware for executing instructions (*e.g*., instructions for carrying out one or more portions of any of the methods disclosed herein), such as those making up a computer program. For example, to execute instructions, the processor 136 may retrieve the instructions from an internal register, an internal cache, the memory 138, or a storage device 140 and decode and execute them. In some examples, the processor 136 may be configured (*e.g*., include programming stored thereon or executed thereby) to carry out one or more portions of any of the example methods disclosed herein.

In some examples, the processor 136 may be configured to perform any of the acts disclosed herein or cause one or more portions of the computing device 134 or controller 112 to perform at least one of the acts disclosed herein. Such configuration can include one or more operational programs (*e.g*., computer program products) that are executable by the at least one processor 136. For example, the processor 136 may be configured to automatically determine a volume of urine in a urine collection container, automatically determine a proximity of urine in the urine collection container to a sensor, automatically transmit an alert when the volume of the urine in the urine collection container meets or exceeds a predetermined threshold, automatically transmit an alert when a fluid is sensed in the fluid collection device 102, determine a difference in fluid intake and fluid output of a user, and/or automatically transmit an alert when a change or recharge of battery is suggested.

The at least one computing device 134 may include at least one memory storage medium (*e.g*., memory 138 and/or storage device 140). The computing device 134 may include memory 148, which is operably coupled to the processor 146. The memory 138 may be used for storing data, metadata, and programs for execution by the processor 136. The memory 138 may include one or more of volatile and non-volatile memories, such as Random Access Memory (RAM), Read Only Memory (ROM), a solid-state disk (SSD), Flash, Phase Change Memory (PCM), or other types of data storage. The memory 138 may be internal or distributed memory.

The computing device 134 may include the storage device 140 having storage for storing data or instructions. The storage device 140 may be operably coupled to the at least one processor 136. In some examples, the storage device 140 may comprise a non-transitory memory storage medium, such as any of those described above. The storage device 140 (e.g., non-transitory storage medium) may include a hard disk drive (HDD), a floppy disk drive, flash memory, an optical disc, a magneto-optical disc, magnetic tape, or a Universal Serial Bus (USB) drive or a combination of two or more of these. Storage device 140 may include removable or non-removable (or fixed) media. Storage device 140 may be internal or external to the computing device 134. In some examples, storage device 140 may include non-volatile, solid-state memory. In some examples, storage device 140 may include read-only memory (ROM). Where appropriate, this ROM may be mask programmed ROM, programmable ROM (PROM), erasable PROM (EPROM), electrically erasable PROM (EEPROM), electrically alterable ROM (EAROM), or flash memory or a combination of two or more of these. In some examples, one or more portions of the memory 138 and/or storage device 140 (e.g., memory storage medium(s)) may store one or more databases thereon.

In some examples, one or more of a history of the volume of the fluid in the fluid collection container 106, a trend of the volume of the fluid in the fluid collection container 106, a history of a fluid collection device 102 replacement, and/or a history of battery 118 replacement or recharging may be stored in a memory storage medium such as one or more of the processor 136 (*e.g.,* internal cache of the processor), memory 138, or the storage device 140.

The computing device 134 also includes one or more I/O devices/interfaces 142, which are provided to allow a user to provide input to, receive output from, and otherwise transfer data to and from the computing device 134. These I/O devices/interfaces 142 may include a touch screen, switches, network interface, web-based access, modem, a port, other known I/O devices or a combination of such I/O devices/interfaces 142. The touch screen may be activated with a stylus or a finger.

The I/O devices/interfaces 142 may include one or more devices for presenting output to a user, including, but not limited to, a graphics engine, a display (*e.g.,* a display screen or monitor), one or more output drivers (*e.g*., display drivers), one or more audio speakers, and one or more audio drivers. In certain examples, I/O devices/interfaces 142 are configured to provide graphical data to a display for presentation to a user. The graphical data may be representative of one or more graphical user interfaces and/or any other graphical content as may serve a particular implementation.

The computing device 134 may further include a communication interface 144. The communication interface 144 may include hardware, software, or both. The communication interface 144 may provide one or more interfaces for communication (such as, for example, packet-based communication) between the computing device 134 and one or more networks. For example, communication interface 144 may include a network interface controller (NIC) or network adapter for communicating with an Ethernet or other wire-based network or a wireless NIC (WNIC) or wireless adapter for communicating with a wireless network, such as a WI-FI, Bluetooth, proximity sensors, etc. As discussed above, in some embodiments, the computing device 134 may communicate with and/or control the operations of the pump 108.

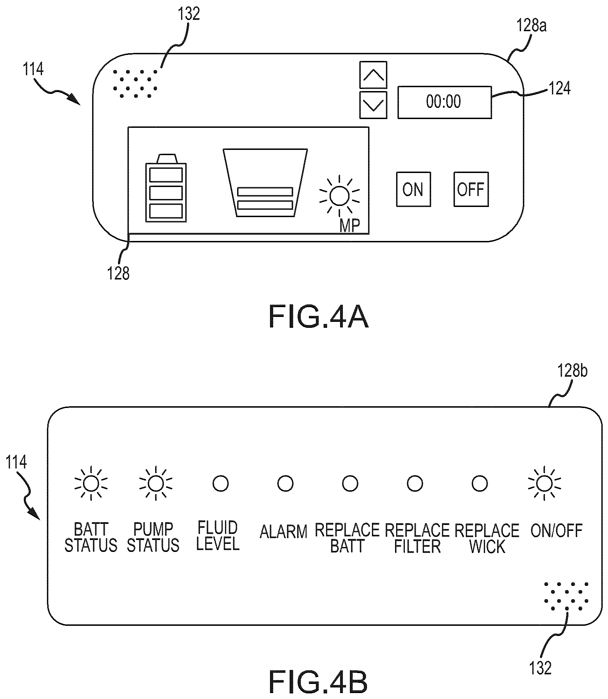

In some embodiments, the computing device 134 may communicate with control panel 114. Referring now to **FIGS. 4A-4B****,** in an embodiment, the controller 112 may be configured to transmit a signal including at least one of a property or status of the fluid collection system 100 to the control panel 114. In some embodiments, the control panel may include display 128a that may include at least one of a property or status of the fluid collection system 100. For example, the display 128a may include one or more of a battery status, a volume of fluid in the fluid collection container 106, an indicator light and/or display character that the pump is running, buttons to turn the fluid collection system 100 on and off, a time since last change of the fluid collection device 102, time since last cleaning of a component of the fluid collection system 100, an amount of fluid intake by the user, a difference in fluid intake by the user and the volume of fluid in the fluid collection container 106, or a system integrity status.

Referring to FIG. 4B, in some embodiments, the display 128b may include at least one indicator light. In some embodiments, the display 128b may include several indicator lights, wherein the status of the light being lit or unlit indicates a status of a component of the fluid collection system 100. In some embodiments, a color of the indicator light may indicate a status of the component. For example, the fluid level indicator light of display 128b may indicate a green light when the fluid collection container 106 is capable of retaining fluid, a yellow light when the fluid collection container 106 is approaching a full level and a red light when the fluid collection container 106 is required to be emptied. As another example, a "replace filter" indicator light may be unlit while the timer 124 indicates the filter does not require replacement, and may be lit when the timer 124 has completed a predetermined cycle to indicate to the user and/or caregiver that the filter should be replaced. A speaker may be included in the control panel 114 to provide an audible alert to indicate a condition that requires that may require the immediate attention of the user or caregiver. For example, a full fluid collection container 106 or an extremely low battery indication.

In some embodiments, the display 128a,b may be configured to provide the at least one of the property or status of the fluid collection system 100 in real-time as communicated by the controller 112. The controller 112 may transmit to the computing device 134 a property or status of the fluid collection system 100 or an alert to the electronic device of the user or the caregiver when a battery powering at least one of the controller 112, the control panel 114, or the pump 108 is low. The controller 112 may transmit an alert when cleaning or replacement of the fluid collection device 102 or other component may be due. In an embodiment, the display 128a,b may provide at least the current status of the battery 118, the fill level of the fluid collection container 106, an input from the user or caregiver to include the volume of fluid consumed by the user, the time since the last replacement or cleaning of the fluid collection device 102, and the time since the least cleaning or replacement of the fluid collection container 106. While an example control panel 114 is shown in **FIGS. 4A-4B****,** the components illustrated in **FIGS. 4A-4B** are not intended to be limiting of the controller 112 or control panel 114. Additional or alternative components may be used in some examples. In some examples, the controller 112 or the control panel 114 may include fewer components than those shown in **FIG. 4A-4B****.**

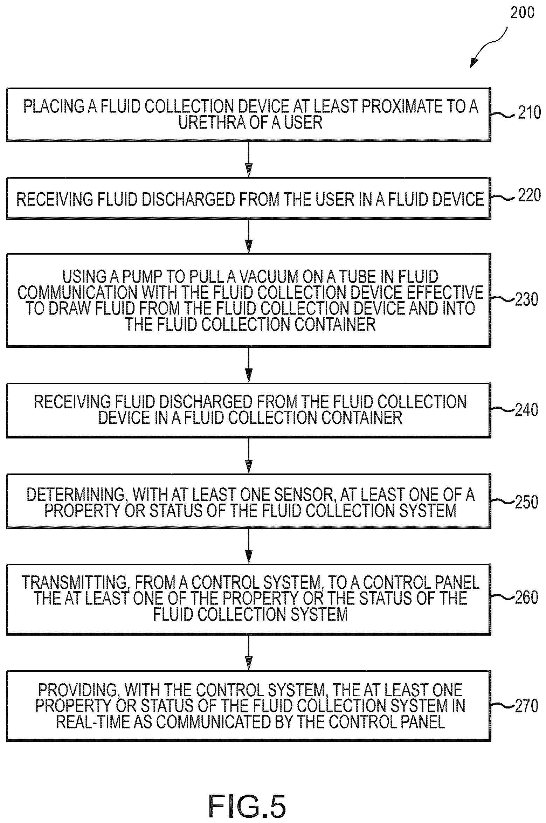

**FIG. 5** is a flow diagram of a method 200 for using a fluid collection system, according to an embodiment. The method 200 includes an act 210 of placing a fluid collection device at least proximate to a urethra of a user and an act 220 of receiving fluid discharged from the user in a fluid collection device. The method 200 includes an act 230 of using a pump to pull a vacuum on a tube in fluid communication with the fluid collection device effective to draw fluid from the fluid collection device and into the fluid collection container.

The method 200 also includes an act 240 of receiving fluid discharged from the fluid collection device in a fluid collection container. The method 200 also includes an act 250 of detecting, with at least one sensor, at least one of a property or status of the fluid collection system. In some embodiments, the at least one sensor may include a fluid level sensor in the fluid collection container. The act 250 may include at least one of a volume of fluid within the fluid collection device or the fluid collection container, a pump status, or a battery status. The method 200 also include act 260 of transmitting, from a control system, to a control panel the at least one of the property or status of the fluid collection system. In an embodiment, method 200 may also include an act 270 of providing, with the control system, the at least one property or status of the fluid collection system in real-time as communicated by the control panel.

The method 200 may include any of the fluid collection systems and/or devices described or incorporated by reference herein. It should be appreciated that the acts of the method 200 described above are for illustrative purposes. For example, the acts of the method 200 can be performed in different orders, split into multiple acts, modified, supplemented, or combined. In an embodiment, one or more of the acts of the method 200 can be omitted from the method 200. Any of the acts of the method 200 can include using any of the portable fluid collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting.

The following clauses describe aspects of the disclosure that may or may not presently be claimed:
1. A fluid collection system, comprising:
   a fluid collection device configured to collect fluid discharged from a user;
   a fluid collection container configured to receive the fluid from the fluid collection device;
   a pump in fluid communication with the fluid collection device and the fluid collection container, wherein the pump is configured to pull an at least partial vacuum to draw the fluid from the fluid collection device and into the fluid collection container; and
   a control system including at least one sensor configured to monitor at least one of a property or status of the fluid collection system, and a controller operatively coupled to the at least one sensor and configured to communicate at least one of the property or the status of the fluid collection system to a control panel.
2. The fluid collection system of clause 1, wherein the at least one sensor includes a fluid level sensor disposed adjacent to the fluid collection container.
3. The fluid collection system of clause 2, wherein the urine level sensor is a capacitive sensor configured to detect a property related to the volume or mass of the fluid in the fluid collection container.
4. The fluid collection system of clause 1, wherein the at least one sensor includes a power consumption sensor operatively coupled to the pump.
5. The fluid collection system of clause 1, further comprising a battery operably coupled to the pump, wherein the control system is configured to communicate a battery status to the control panel.
6. The fluid collection system of clause 1, wherein the control system is configured to control the pump.
7. The fluid collection system of any of the preceding clauses, wherein the pump is disposed in a housing configured to couple to the fluid collection container.
8. The fluid collection system of clause 7, wherein the control panel is located on the housing or on a remote controller.
9. The fluid collection system of any of the preceding clauses, wherein the control panel includes at least one status indicator.
10. The fluid collection system of clause 9, wherein the status indicator includes at least one of a digital display or an indicator light.
11. The fluid collection system of clause 9, wherein the status indicator includes an audible indication.
12. The fluid collection system of any of the preceding clauses, wherein the control panel includes a user input to the control system.
13. The fluid collection system of any of the preceding clauses, wherein the control system includes at least one of programmable timer, an alert or alarm, or a display configured to display the at least one of the property or the status of the fluid collection system.
14. The fluid collection system of clause 13, wherein the programmable timer is configured to indicate when a component of the fluid collection system requires replacement.
15. The fluid collection system of any of clauses 1-14, wherein the control system is configured to provide the at least one of the property or the status of the fluid collection system in real-time as communicated by the control panel.
16. A method of using a fluid collection system, the method comprising:
   placing a fluid collection device at least proximate to a urethra of a user;
   receiving fluid discharged from the user in a fluid collection device;
   receiving fluid discharged from the fluid collection device in a fluid collection container;
   determining, with at least one sensor, at least one of a property or status of the fluid collection system; and
   transmitting, from a control system, to a control panel the at least one of the property or the status of the fluid collection system.
17. The method of clause 16, further comprising using a pump to pull a vacuum on a tube in fluid communication with the fluid collection device effective to draw fluid from the fluid collection device and into the fluid collection container.
18. The method of clause 16 or 17, wherein determining with at least one sensor at least one of a property or status of the fluid collection system includes detecting at least one of a volume of fluid within the fluid collection device or the fluid collection container, a pump status, or a battery status.
19. The method of any of clauses 16-18, wherein transmitting, from a control system, to a control panel the at least one of the property or the status of the fluid collection system includes transmitting to a control panel located on a housing for the pump or on a remote controller.
20. The method of any of clauses 16-19, further comprising providing, with the control system, the at least one property or status of the fluid collection system in real-time as communicated by the control panel.

## Claims

1. A fluid collection system (100), comprising:
a fluid collection device (102) configured to collect fluid discharged from a user;
a fluid collection container (106) configured to receive the fluid from the fluid collection device;
a pump (108) in fluid communication with the fluid collection device and the fluid collection container, wherein the pump is configured to pull an at least partial vacuum to draw the fluid from the fluid collection device and into the fluid collection container; and
a control system (111) including at least one sensor (110,116,122) configured to monitor at least one of a property or status of the fluid collection system, and a controller operatively coupled to the at least one sensor and configured to communicate at least one of the property or the status of the fluid collection system to a control panel (114);
wherein the at least one sensor includes a fluid level sensor (116) within and/or adjacent the fluid collection container (106);
wherein, based on data from the fluid level sensor, the controller is configured to start the pump (108) to cause fluid to flow from the fluid collection device (102) to the fluid collection container (106) and to stop the pump (108) when the volume of fluid in the fluid collection container (106) reaches a predetermined threshold.

2. The fluid collection system of claim 1, wherein the fluid level sensor is a capacitive sensor configured to detect a property related to the volume or mass of the fluid in the fluid collection container.

3. The fluid collection system of claim 1, wherein the at least one sensor includes a power consumption sensor (122) operatively coupled to the pump.

4. The fluid collection system of claim 1, further comprising a battery operably coupled to the pump, wherein the control system is configured to communicate a battery status to the control panel.

5. The fluid collection system of any of the preceding claims, wherein the pump is disposed in a housing configured to couple to the fluid collection container; and optionally wherein the control panel is located on the housing or on a remote controller.

6. The fluid collection system of any of the preceding claims, wherein the control panel includes at least one status indicator.

7. The fluid collection system of claim 6, wherein the status indicator includes at least one of a digital display or an indicator light; or wherein the status indicator includes an audible indication.

8. The fluid collection system of any of the preceding claims, wherein the control panel includes a user input to the control system.

9. The fluid collection system of any of the preceding claims, wherein the control system includes at least one of programmable timer, an alert or alarm, or a display (128) configured to display the at least one of the property or the status of the fluid collection system; and optionally wherein the programmable timer is configured to indicate when a component of the fluid collection system requires replacement.

10. The fluid collection system of any of claims 1-9, wherein the control system is configured to provide the at least one of the property or the status of the fluid collection system in real-time as communicated by the control panel.

11. A method of using a fluid collection system (100), the method comprising:
placing a fluid collection device (102) at least proximate to a urethra of a user;
receiving fluid discharged from the user in the fluid collection device;
receiving fluid discharged from the fluid collection device in a fluid collection container (106);
determining, with at least one sensor (110,116,122) , at least one of a property or status of the fluid collection system; wherein the at least one sensor includes a fluid level sensor (116) within and/or adjacent the fluid collection container (106); and
transmitting, from a control system (111), to a control panel (114) the at least one of the property or the status of the fluid collection system,
wherein, based on data from the fluid level sensor (116), the controller starts a pump (108) to cause fluid to flow from the fluid collection device (102) to the fluid collection container (106) and stops the pump (108) when the volume of fluid in the fluid collection container (106) reaches a predetermined threshold.

12. The method of claim 11, further comprising using the pump to pull a vacuum on a tube in fluid communication with the fluid collection device effective to draw fluid from the fluid collection device and into the fluid collection container.

13. The method of claim 11 or 12, wherein determining with at least one sensor at least one of a property or status of the fluid collection system includes detecting at least one of a volume of fluid within the fluid collection device or the fluid collection container, a pump status, or a battery status.

14. The method of any of claims 11-13, wherein transmitting, from a control system, to a control panel the at least one of the property or the status of the fluid collection system includes transmitting to a control panel located on a housing for the pump or on a remote controller.

15. The method of any of claims 11-14, further comprising providing, with the control system, the at least one property or status of the fluid collection system in real-
time as communicated by the control panel.
